# EUROPÄISCHE PATENTSCHRIFT

(11) **EP 0 671 149 B1**
(45) Veröffentlichungstag und Bekanntmachung des Hinweises auf die Patenterteilung: **15.11.2000**
(21) Anmeldenummer: 95101972.8
(22) Anmeldetag: 14.02.1995
(51) Int. Cl.: A61B 17/11

(54) **Chirurgisches Anastomosenring-Setzgerät**
Device for placing an anastomosis ring
Dispositif de pose d'un anneau d'anastomose

(30) Priorität: 09.03.1994 DE 4407668
(43) Veröffentlichungstag der Anmeldung: 13.09.1995
(73) Patentinhaber: Köckerling, Ferdinand, Dr., D-91054 Erlangen (DE); Schneider, Ignaz, Dr., D-91301 Forchheim (DE)
(72) Erfinder: Köckerling, Ferdinand, Dr., D-91054 Erlangen (DE); Schneider, Ignaz, Dr., D-91301 Forchheim (DE)
(74) Vertreter: Rau, Manfred, Dr. Dipl.-Ing.

(56) Entgegenhaltungen:
- EP-A- 0 152 382
- EP-A- 0 349 197
- EP-A- 0 564 783
- EP-A- 0 568 774
- US-A- 4 245 638
- US-A- 4 505 272

## Beschreibung

Die Erfindung betrifft ein chirurgisches Anastomosenring-Setzgerät zur Verwendung bei der offenen oder laparoskopischen Darmchirurgie.

Bei der Entfernung von beispielsweise tumorverseuchten Darmabschnitten besteht das Problem die offenen Darmenden miteinander zu verbinden. Dieses Problem wird üblicherweise mit Hilfe sogenannter Anastomosenringe gelöst, wie sie beispielsweise unter dem Warenzeichen "VALTRAC"^{(R)} handelsüblich sind. Solche biofragmentierbaren Anastomosenringe bestehen aus bei pilzförmigen Teilen deren Schäfte teleskopartig ineinander verschiebbar geführt sind und eine Durchflußöffnung für den Darminhalt bilden. Die etwa halbkugelförmigen Köpfe dieser Teile weisen in einander entgegengesetzte Richtungen. Ihre aufeinanderzuweisenden Randkanten können durch Zusammenschieben der beiden Teile und deren Verrastung in der zusammengeschobenen Stellung die beiden offenen Enden des Darmlumens miteinander verbinden. Dazu werden die beiden offenen Enden jeweils mit einer sogenannten Tabaksbeutelnaht versehen die beiden pilzförmigen Köpfe des Anastomosenrings jeweils in die beiden offenen Darmlumen eingeführt, mit Hilfe der Tabaksbeutelnaht die offenen Enden straff über die Schäfte des Ringes zusammengezogen sowie die beiden Teile zusammengeschoben und verrastet. Damit ist eine feste Verbindung zwischen den beiden offenen Darmlumen geschaffen wobei sich durch die Biofragmentierbarkeit des Ringmaterials dieser innerhalb eines Zeitraumes von etwa 2 bis 3 Wochen auflöst und abgestoßen wird. In dieser Zeit können die beiden offenen Enden miteinander verwachsen.

Nachteilig bei dieser bekannten Vorgehensweise zum Setzen eines Anastomosenringes ist die Tatsache, daß die Ringteile in ihrer eingesetzten Stellung von außen durch Zugriff über die Darmwand zusammengeschoben und verrastet werden müssen.

Damit ist diese Operationstechnik bei der laparoskopischen Chirurgie oder - falls beengte Platzverhältnisse, wie beispielsweise bei Mastdarmoperationen, herrschen - auch bei der offenen Chirurgie nicht anwendbar.

Ein chirurgisches Anastomosenring-Setzgerät, mit dessen Hilfe die vorstehende Operationstechnik auch in der laparoskopischen Chirurgie oder bei beengten Platzverhältnissen auch in der offenen Chirurgie anwendbar wird, ist aus der EP-A-0 564 783 bzw. EP-A-0 568 774 bekannt. Demnach ist das Setzgerät mit einem in den Darmtrakt vorzugsweise rektal einführbaren Einführungsrohr, einem am körperinneren Ende des Einführungsrohres angeordneten, zylindrischen Haltekopf für einen Anastomosenring, einer im Bereich des Haltekopfes angeordneten Schiebermechanik zum Zusammenschieben und Verrasten des Anastomosenringes und einem am körperäußeren Ende des Einführungsrohres angeordneten Antrieb für die Schiebermechanik versehen. Die Schiebermechanik ist dabei über ein Getriebe vom körperäußeren Ende des Einführungsrohres her betätigbar.

Durch die vorstehend geschilderte Ausgestaltung des Anastomosenring-Setzgerätes entfällt die Notwendigkeit, das Zusammenschieben und Verrasten des Ringes durch eine direkte Manipulation des Ringes von der Außenseite der zu verbindenden Darmabschnitte her vorzunehmen. Der Ring kann also an verborgener oder schlecht zugänglicher Stelle plaziert werden.

Der Erfindung liegt die Aufgabe zugrunde, ein chirurgisches Anastomosenring-Setzgerät zu schaffen, das insbesondere im Bereich der Schiebermechanik im Haltekopf hinsichtlich der Zuverlässigkeit der Steuerung der Schiebermechanik und deren Kompaktheit verbessert ist.

Diese Aufgabe wird durch das im Anspruch 1 angegebene Anastomosenring-Setzgerät gelöst.

Weitere Merkmale, Einzelheiten und vorteilhafte Ausführungsformen des erfindungsgemäßen Anastomosenring-Setzgerätes ergeben sich aus den Unteransprüchen und der nachfolgenden Beschreibung, in der ein Ausführungsbeispiel des Erfindungsgegenstandes anhand der beigefügten Figuren näher erläutert wird. Es zeigen:
- Fig. 1: eine Seitenansicht eines erfindungsgemäßen Anastomosenring-Setzgerätes in teilweise geschnittener Darstellung,
- Fig. 2: eine Seitenansicht des Kopfbereiches des Setzgerätes aus Pfeilrichtung II nach Fig. 1,
- Fig. 3: eine Draufsicht des Setzgerätes aus Pfeilrichtung III nach Fig. 2
- Fig. 4: eine Seitenansicht des Kopfsockels des Setzgerätes,
- Fig. 5: eine Seitenansicht des Kopfgehäuses des Setzgerätes,
- Fig. 6 und 7: zwei unterschiedliche Seitenansichten des Steuerschiebers,
- Fig. 8 und 9: zwei unterschiedliche Seitenansichten eines Mitnehmerhebels,
- Fig. 10 und 11: zwei unterschiedliche Seitenansichten des Lagerschiebers des Setzgerätes nach Fig. 1 und
- Fig. 12 bis 14: ein Ablaufschema der Mitnehmer-Betätigung des Setzgerätes nach Fig. 1.

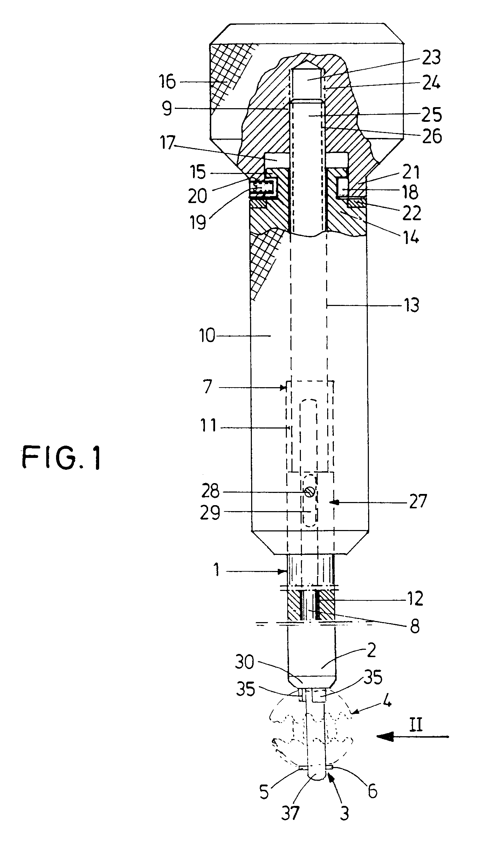

Wie aus Fig. 1 deutlich wird, weist ein erfindungsgemäßes Anastomosenring-Setzgerät als wesentliches Bauteil ein in den Darmtrakt rektal einführbares Einführungsrohr 1 auf, das gerade oder auch gekrümmt sein kann. Am körperinneren Ende 2 des Einführungsrohres 1 ist ein im wesentlicher zylindrischer Haltekopf 3 für einen üblichen VALTRAC ^{(R)}-Anasatomosenring 4 angeordnet. Innerhalb des Haltekopfes 3 ist eine noch näher zu beschreibende Schiebermechanik zum Zusammenschieben und Verrasten des Anastomoseminges 4 angebracht, von der in Fig. 1 die beiden seitlichen Mitnehmer 5,6 erkennbar sind. Die Schiebermechanik ist vom körperäußeren Ende 7 des Einführungsrohres 1 her über ein Getriebe betätigbar, das mit einem am körperäußeren Ende 7 des Einführungsrohres 1 angeordneten Antrieb versehen ist.

Getriebe und Antrieb für die Schiebermechanik sind durch eine im Einführungsrohr 1 verlaufende Schubstange 8 mit Spindeltrieb 9 wie folgt gebildet.

Auf das körperäußere Ende 7 des Einführungsrohres 1 ist koaxial ein im wesentlichen zylindrisches Griffstück 10 mittels der Schraubverbindung 11 aufgeschraubt, wobei das Griffstück 10 eine koaxial mit der Rohröffnung 12 verlaufende, durchgehende Innenbohrung 13 aufweist. An dem dem Einführungsrohr 1 abgewandten Stirnende 14 des Griffstückes 1o ist eine ebenfalls koaxial verlaufende Ringschulter 15 vorgesehen, auf der ein Spindelrad 16 mit seiner zylindrischen Innenöffnung 17 drehbar gelagert ist. Am äußeren Umfang der Ringschulter 15 ist ferner eine Umfangsnut 18 vorgesehen, in die von außen Madenschrauben 19 als Vorsprünge eingreifen. Diese Madenschrauben 19 sind durch entsprechende Gewindebohrungen 20 in dem Ringkragen 21 um die zylindrische Innenöffnung 17 des Spindelrades 16 durchgeschraubt und legen letzteres in längsaxialer Richtung des Einführungsrohres 1 fest. Zwischen der Stirnseite des Ringkragens 21 und dem Griffstück 10 ist ein Gleitring 22 aus Messing eingesetzt.

Das Spindelrad 16 bildet mit seiner koaxial zur Längsachse A des Einführungsrohres 1 angeordneten, von der Innenöffnung 17 ausgehenden Sacklochbohrung 23 mit Innengewinde 24 zusammen mit einem Gewindebolzen 25 in der Innenbohrung 13 des Griffstückes 10 einen Spindeltrieb 9 für die Schiebermechanik. Dazu steht das Außengewinde 26 des Gewindebolzens 25 mit dem Innengewinde 24 im Spindelrad 16 in Eingriff Weiterhin ist der Gewindebolzen 25 an seinem dem Spindelrad 16 abgewandten Ende 27 mit der Schubstange 8 verbunden, die zur Schiebermechanik am körperinneren Ende 2 des Einführungsrohres 1 führt. Aufgrund der vorstehend beschriebenen Konstruktion bilden der Gewindebolzen 25 gemeinsam mit der Schubstange 8 ein Getriebe zur Übertragung der vom Spindelrad 16 herrührenden Drehantriebsbewegung auf die Schiebermechanik. Dabei ist ferner die Einheit aus Gewindebolzen 25 und Schubstange 8 in Drehrichtung arretiert. Für diese Dreharretierung ist der Gewindebolzen 25 auf seiner Außenseite mit einer radial abstehenden Erhebung in Form eines Schrauberkopfes 28 einer radial in den Gewindebolzen eingeschraubten Schraube versehen, welcher Schraubenkopf 28 in ein parallel zur Längsachse A des Einführungsrohres 1 darin verlaufendes Langloch 29 eingreift.

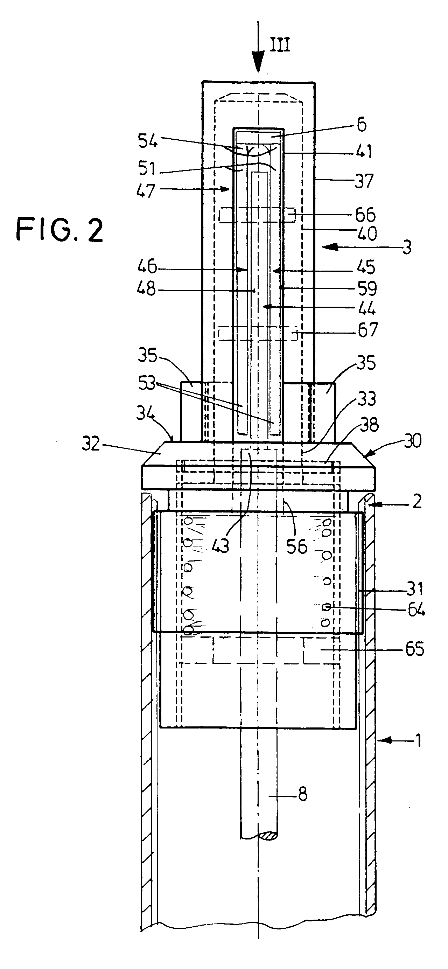

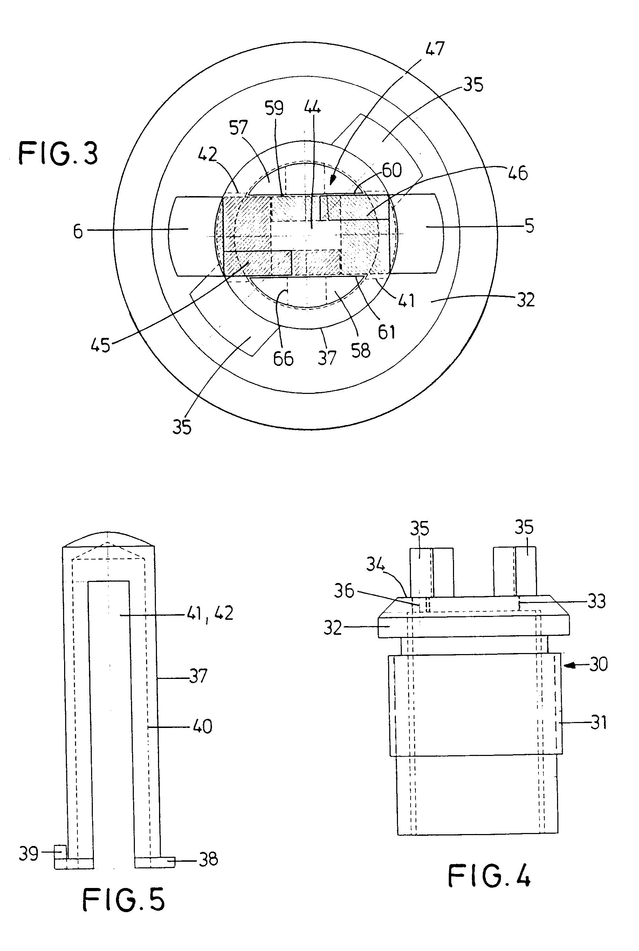

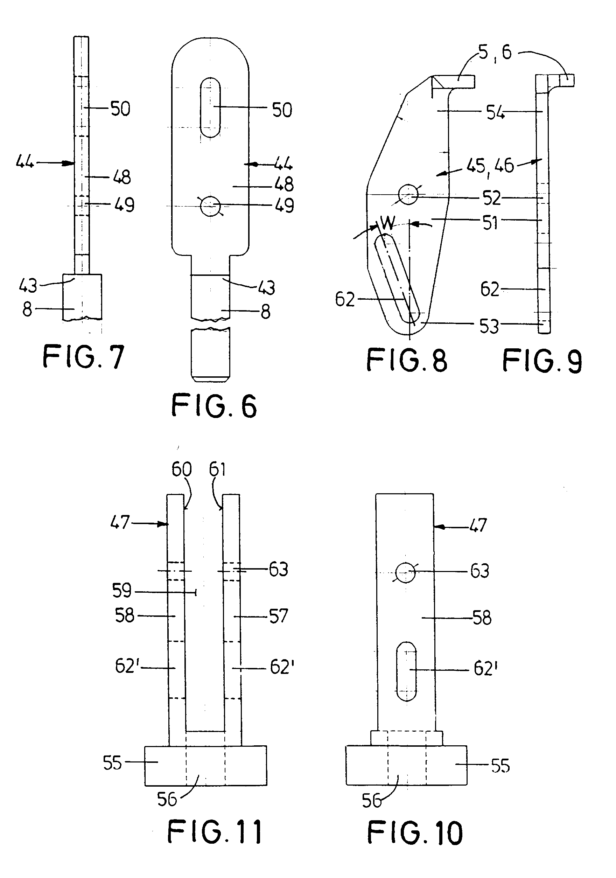

Anhand der Fig. 2 ist der grundsätzliche Aufbau des Haltekopfes 3 zu erläutern. Dieser weist einerseits einen im wesentlichen zylindrischen Kopfsockel 30 auf, der mit seinem etwa hohlzylindrischen Gewindeteil 31 in die Innenöffnung am körperinneren Ende 2 des Einführungsrohres 1 eingeschraubt ist. Ein ringförmiger Kragen 32 liegt dabei dicht an der Stirnkante des Einführungsrohres 1 an. Der Kopfsockel 30 ist ferner mit einer koaxialen Durchgangsöffnung 33 versehen, die auf der Stirnseite 34 des Kragens 32 von zwei diametral gegenüberliegenden Zentrieransätzen 35 für den Anastomosenring 4 umgeben ist. Seitlich neben der Durchgangsöffnung 33 (Fig. 4) sitzt am Boden des Gewindeteils 31 eine kleine axialparallel verlaufende Bohrung 36. Diese dient zur Dreharretierung des Kopfgehäuses 37 (Fig. 5), das von der Seite des Gewindeteils 31 her durch die Durchgangsöffnung 33 durchgesteckt ist, bis sein Kragen 38 am unteren Ende auf der Innenseite des Kragens 32 des Kopfsockels 30 anschlägt. Ein Stift 39 auf der Oberseite des Kragens 38 greift dabei zur besagten Dreharretierung in die Bohrung 36 ein.

Das Kopfgehäuse 37 ist kappenförmig ausgestaltet und weist eine axialparallele, zylindrische Lagerbohrung 40 sowie ebenfalls axialparallele, radial gegenüberliegende, in Seitenansicht (Fig. 5) langgestreckt rechteckige Wandöffnungen 41,42 zur Aufnahme der noch zu erläuternden Schiebermechanik auf. Im übrigen sind die Wandöffnungen 41,42 im Bereich des Kragens 38 offen.

Die Schiebermechanik weist einen einstückig an das körperinnere Ende 43 der Schubstange 8 angeformten Steuerschieber 44 (Fig. 6,7), zwei Mitnehmerhebel 45,46 (Fig. 8,9) sowie einen Lagerschieber 47 (Fig. 10,11) auf.

Der Steuerschieber 44 ist als ebener, im wesentlichen rechteckiger Plattenkörper 48 ausgestaltet, der in axialparalleler Richtung hintereinanderliegend eine Lagerbohrung 49 sowie ein axialparallel verlaufendes Langloch 50 aufweist. Die beiden doppelarmigen Mitnehmerhebel 45,46 bestehen aus einem etwa rautenförmigen Plattenkörper 51, der mit einer zentral angeordneten Schwenklagerbohrung 52 und im Bereich des hinteren Endes 53 mit einem im spitzen Winkel W zur Längsachse A verlaufenden Langloch 62 versehen ist. Am zweiten Ende 54 des Platterkörpers 51 ist jeweils der seitlich und rechtwinklig davon abstehende Mitnehmer 5,6 angeformt (Fig. 8,9).

Der Lagerschieber 47 (Fig. 10,11) besteht aus einem zylindrischen Sockel 55, der mit einer rotationssymetrischen Durchgangsöffnung 56 versehen ist, die zwischen den beiden axialparallelen Gabelschenkeln 57,58 des Lagerschiebers 47 mündet. Die beiden Gabelschenkel 57,58 bilden gemeinsam einen zylindrischen Außenumriß und zwischen sich den Aufnahmespalt 59, der von den ebenen Innenwänden 60,61 der Gabelschenkel 57,58 begrenzt ist. In diesen Gabelschenkeln 57,58 sind dem Sockel 55 zugewandt ein axialparalleles Langloch 62', sowie etwa mittig zwischen dem Langloch 62' und dem freien Ende der Gabelschenkel 57,58 eine weitere Schwenklagerbohrung 63 vorgesehen.

Steuerschieber 44, Mitnehmerhebel 45,46 und Lagerschieber 47 sind wie folgt im Haltekopf 3 angeordnet (Fig. 2,3):
Der Lagerschieber 47 ist von der Seite des Einführungsrohres 1 her in die Lagerbohrung 40 des Kopfgehäuses 37 eingeschoben, bis sein Sockel 55 am Kragen 38 des Kopfgehäuses 37 zu liegen kommt. Vor dem Sockel 55 liegt dabei eine Schraubendruckfeder 64, die sich auf einem in das Gewindeteil 31 des Kopfsockels 30 eingeschraubten Gegenlager 65 abstützt. Damit ist der Lagerschieber 47 entgegen der Mitnahmerichtung M bezüglich des Anastomosenringes 4 federbeaufschlagt.

Die Gabelschenkel 57,58 des Lagerschiebers 47 sitzen innerhalb der Lagerbohrung 40 des Kopfgehäuses 37 und sind so ausgerichtet, daß der Aufnahmespalt 59 mit den Wandöffnungen 41,42 des Kopfgehäuses 37 fluchtet. Durch die Durchgangsöffnung 56 des Lagerschiebers 47 verläuft die Schubstange 8 derart, daß der daran angeformte Steuerschieber 44 mittig zwischen den beiden Gabelschenkeln 57,58 liegt. Beiderseits des Steuerschiebers 44 ist jeweils einer der beiden Mitnehmerhebel 45,46 in den Aufnahmespalt 59 eingesetzt, wobei die Hauptebenen des Steuerschiebers 44 und der Mitnehmerhebel 45,46 zueinander parallel und parallel zu den Innenwänden 60,61 der Gabelschenkel 57,58 verlaufen.

Die beiden Mitnehmerhebel 45,46 sind über einen Schwerkbolzen 66, der jeweils ihre Schwerklagerbohrungen 52 durchsetzt und dessen Enden in den gegenüberliegenden Schwerklagerbohrungen 63 des Lagerschiebers 47 sitzen, schwenkbar am Lagerschieber 47 gelagert. Dieser Schwenkbolzen 66 durchgreift das Langloch 50 im Steuerschieber 44.

In die Lagerbohrung 49 des Steuerschiebers 44 ist ferner ein beiderseits überstehender Steuerbolzen 67 eingesetzt, der in den Langlöchern 62,62' in den Mitnehmerhebeln 45,46 bzw. im Lagerschieber 47 verläuft. Die Langlöcher 50, 62 und 62' im Steuerschieber 44 bzw. in den Mitnehmerhebeln 45,46 bzw. im Lagerschieber 47 dienen als Steuerkulissen zur Schwenk- und Verschiebesteuerung der Mitnehmerhebel 45,46.

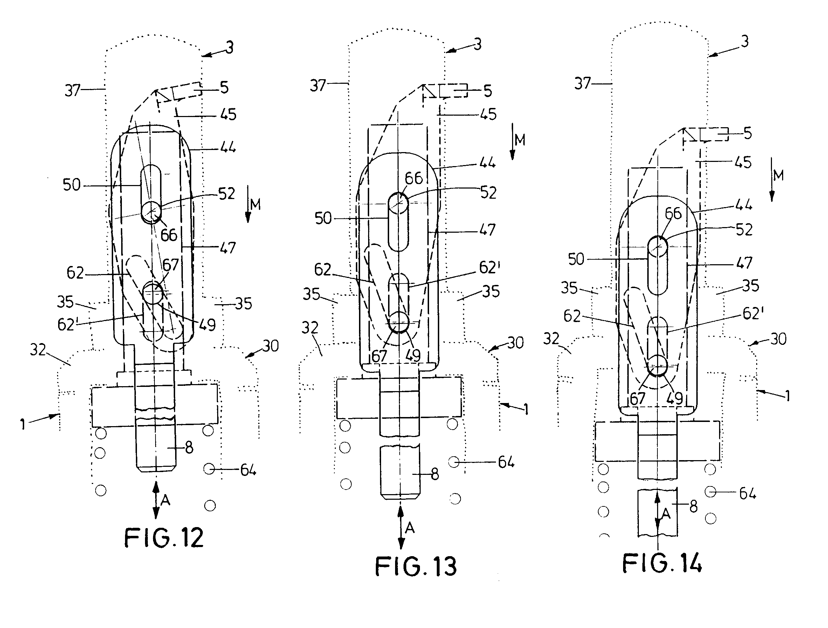

Die Funktionsweise des erfindungsgemäßen Setzgerätes ist anhand der Fig. 12 bis 14 wie folgt zu erläutern, wobei darauf hinzuweisen ist, daß in diesen Zeichnungen aus Übersichtlichkeitsgründen lediglich ein Mitnehmerhebel 45 dargestellt ist. Der zweite Mitnehmerhebel 46 liegt spiegelsymetrisch bezüglich der Längsachse A zum ersten Mitnehmerhebel 45 und vollführt synchron und bezüglich der radialen Ausschwerkung der Mitnehmer gegengleich dieselben Bewegungen.

In Fig. 12 ist die Aufnahmestellung des Setzgerätes dargestellt, in der der Mitnehmerhebel 45 (kurzstrichliert dargestellt) radial nach innen geschwenkt steht, so daß der Mitnehmer 5 innerhalb des Umrisses (punktiert dargestellt) des Kopfgehäuses 37 liegt. In dieser Stellung kann ein Anastomosenring 4 mit seiner Innenöffnung auf den vom Kopfgehäuse 37 gebildeten Schaft aufgeschoben und mit Hilfe der Zentrieransätze 35 in Drehrichtung zentriert und festgelegt werden. Anschließend wird das Spindelrad 16 betätigt, so daß die Schubstange 8 in Richtung zum körperäußeren Ende 7 und damit der Steuerschieber 44 (durchgezogen dargestellt) in Mitnahmerichtung M gezogen werden. Durch die Kulissensteuerung zwischen Steuerbolzen 67 und dem schräggestellten Langloch 62 im Mitnehmerhebel 45 wird dieser bezogen auf Fig. 12 im Uhrzeigersinn um den Schwenkbolzen 66 verschwenkt. Dabei bildet der Schwenkbolzen 66 einen festen Drehpunkt für den Mitnahmehebel 45, da er durch den federbeaufschlagten Lagerschieber 47 (langstrichliert dargestellt) in der in Fig. 12 gezeigten Stellung festgehalten wird. Durch die Relativverschiebung des Steuerschiebers 44 gegenüber dem Lagerschieber 47 und dem Mitnehmerhebel 45 durchläuft der Schwenkbolzen 66 das Langloch 50 im Steuerschieber 44.

In Fig. 13 ist die ausgeschwenkte Stellung des Mitnehmerhebels 45 gezeigt, in der der Mitnehmer 5 über den Außenumriss des Gehäuses 37 hinausteht und den Anastomosenring 4 hintergreifen kann. In dieser Stellung liegt der Steuerbolzen 67 am unteren Ende des Langloch 62' des Lagerschiebers 47 sowie am unteren Ende des schräggestellten Langlochs 62 im Mitnehmerhebel 45 an. Ferner ist der Schwenkbolzen 66 im Langloch 50 des Steuerschiebers 44 an das obere Ende des Langloches 50 gewandert. Bei einer weiteren Verschiebung der Schubstange 8 in Richtung zum körperäußeren Ende 7 hin nimmt der Steuerbolzen 67 des Steuerschiebers 44 einerseits den Lagerschieber 47 entgegen der Beaufschlagung durch die Schraubendruckfeder 64 in diese Richtung mit. In einer synchronen Bewegung wird der Schwenkbolzen 66 durch das obere Ende des Langlochs 50 im Steuerschieber 44 in die gleiche Richtung mitgenommen, so daß Steuerschieber 44, Lagerschieber 47 und insbesondere der Mitnehmerhebel 45 in Richtung zum körperäußeren Ende 7 verschoben werden. Dadurch beaufschlagt der Mitnehmer 5 den Anastomosenring 4 und schiebt dessen beiden Teile zusammen, bis die beiden Teile des Anastomosenringes miteinander verrasten und die Verbindung zwischen den beiden Darmabschnitten hergestellt ist (Fig. 14).

Bei einer gegenläufigen Betätigung des Spindelrades 16 wird die Schubstange 8 mit Steuerschieber 44 wieder in die Gegenrichtung verschoben, womit der Lagerschieber 47 und damit der Mitnehmerhebel 45 unter dem Einfluß der Schraubendruckfeder 64 wieder in die in Fig. 13 gezeigte Stellung verschoben werden. Bei einer weiteren Verschiebung der Schubstange 8 und des Steuerschiebers 44 wird der Mitnehmerhebel 45 wieder radial nach innen geschwenkt, bis die in Fig. 12 gezeigte Stellung eingenommen wird. In dieser Stellung kann der Haltekopf 3 vom im Darm plazierten Anastomosenring 4 abgezogen und das Setzgerät aus dem Darmtrakt entfernt werden.

## Patentansprüche

1. Chirurgisches Anastomosenring-Setzgerät mit:
- einem in den Darmtrakt einführbaren Einführungsrohr (1),
- einem am körperinneren Ende (2) des Einführungsrohres (1) angeordneten, zylindrischen Haltekopf (3) für einen Anastomosenring (4),
- einer im Bereich des Haltekopfes (3) angeordneten Schiebermechanik zum Zusammenschieben und Verrasten des Anastomosenringes (4), die vom körperäußeren Ende (7) des Einführungsrohres (1) her über ein Getriebe (9) betätigbar ist, und
- einem am körperäußeren Ende (7) des Einführungsrohres (1) angeordneten Antrieb (16) für das Getriebe (9) der Schiebermechanik, wobei
die Schiebermechanik mindestens einen Mitnehmer (5,6) aufweist, der in zwei getrennten Betätigungsschritten einerseits aus einer in den Haltekopf (3) zurückgezogenen Stellung in eine aus dem Haltekopf (3) seitlich vorstehenden Stellung radial und andererseits in seiner vorstehenden Stellung unter Mitnahme des Anastomosenrings (4) in dessen verrastete Stellung axial verschiebbar ist, dadurch gekennzeichnet, daß jeweils ein Mitnehmer (5,6) an zwei radial in entgegengesetzter Richtung aus dem Haltekopf (3) ausschwenkbaren und längsaxial verschiebbaren Mitnehmerhebeln (45,46) angeordnet ist, die in einem Lagerschieber (47) verschwenkbar gelagert sind, wobei Mitnehmerhebel (45,46) und Lagerschieber (47) mit einem mit dem Getriebe (9) verbundenen Steuerschieber (44) über eine Steuerkulissenanordnung (50,62,62') gekoppelt sind.

2. Setzgerät nach Anspruch 1, dadurch gekennzeichnet, daß Antrieb und Getriebe für die Schiebermechanik als Spindeitrieb (9) mit einem längsaxial bezüglich der Rohrachse (Längsachse A) festgelegten, koaxial dazu drehbar gelagerten Spindelrad (16) und einer in Drehrichtung arretierten, längsaxial im Einführungsrohr (1) verschiebbaren Schubstange (8) ausgebildet sind, die mit einem Außengewinde (26) an ihrem äußeren Ende (27) mit einem Innengewinde (24) des Spindelrades (16) in Eingriff steht und die mit ihrem körperinneren Ende (43) mit der Schiebermechanik gekoppelt ist.

3. Setzgerät nach Anspruch 1 oder 2, dadurch gekennzeichnet, daß der relativ zum Haltekopf (3) und Steuerschieber (44) axial verschiebbare Lagerschieber (47) entgegen der Mitnahmerichtung (M) des Mitnehmers (5,6) federbeaufschlagt (Schraubendruckfeder 64) ist.

4. Setzgerät nach Anspruch 3, dadurch gekennzeichnet, daß die Steuerkulissenanordnung einerseits eine im spitzen Winkel (W) zur Längsachse (A) des Einführungsrohres (1) verlaufende, langlochförmige Steuerkulisse (62) in jedem Mitnehmerhebel (45,46) und andererseits eine axialparallel verlaufende, langlochförmige Steuerkulisse (62') im Lagerschieber (47) aufweist, in welche Steuerkulissen (62,62') ein im Steuerschieber (44) sitzender Steuerbolzen (67) eingreift.

5. Setzgerät nach einem der vorgenannten Ansprüche, dadurch gekennzeichnet, daß die Steuerkulissenanordnung eine axialparallel verlaufende, langlochförmige Steuerkulisse (50) im Steuerschieber (44) aufweist, durch die ein Schwenkbolzen (66) zur Schwenklagerung der Mitnehmerhebel (45, 46) am Lagerschieber (47) greift.

6. Setzgerät nach einem der vorgenannten Ansprüche, dadurch gekennzeichnet, daß der Steuerschieber (44) einstückig an das körperinnere Ende (43) der Schubstange (8) angeformt ist.

7. Setzgerät nach einem der vorgenannten Ansprüche, dadurch gekennzeichnet, daß der Lagerschieber (47) gabelförmig ausgebildet ist, zwischen dessen beiden Gabelschenkeln (57,58) der Steuerschieber (44) und die Mitnehmerhebel (45,46) angeordnet sind.

8. Setzgerät nach einem der vorgenannten Ansprüche, dadurch gekennzeichnet, daß der Haltekopf (3) ein kappenförmiges Kopfgehäuse (37) aufweist, das eine axialparallele, zylindrische Lagerbohrung (40) mit axialparallelen, radial gegenüberliegenden Wandöffnungen (41,42) zur Aufnahme von Steuerschieber (44), Lagerschieber (47) und Mitnehmerhebeln (45,46) aufweist.

## Claims

1. A surgical anastomosis ring insertion device comprising:
- a leading-in tube (1) insertable into the intestinal tract;
- a cylindrical anastomosis ring (4) retaining head (3) disposed on the distal end (2) of the leading-in tube (1);
- a slider mechanism, disposed in the vicinity of the retaining head (3), for engaging and locking the anastomosis ring (4), which slider mechanism is actuatable by way of a transmission (spindle drive 9) from the proximal end (7) of the leading-in tube (1); and
- a drive (spindle wheel 16), disposed on the proximal end (7) of the leading-in tube (1), for the transmission (spindle drive 9) of the slider mechanism, the slider mechanism having at least one driver (5, 6), which is displaceable in two operational steps, i.e. on the one hand it is radially displaceable from a position retracted into the retaining head (3) into a position laterally projecting from the retaining head (3), and on the other hand, in its projecting position, while driving the anastomosis ring (4), it is axially displaceable into the position of locking thereof;
characterized in that in each case one driver (5, 6) is disposed on two driving levers (45, 46), which are pivotal out of the retaining head (3) radially in opposite direction and longitudinally axially displaceable and which are pivotally positioned in a bearing slider (47), the driving levers (45, 46) and the bearing slider (47) being coupled, by way of a driving link arrangement (50, 62, 62'), with a control slider (44) connected with the transmission (9).

2. An insertion device according to claim 1, characterized in that the drive and the transmission for the slider mechanism are designed as a spindle drive (9), which comprises a spindle wheel (16) arrested longitudinally axially in relation to the tube axis (longitudinal axis A) and rotatably supported coaxially thereto and a push bar (8), which is arrested in the direction of rotation and displaceable longitudinally axially in the leading-in tube (1) and of which an external thread (26) on its outer end (27) is in engagement with an internal thread (24) of the spindle wheel (16) and of which the distal end (43) is coupled with the slider mechanism.

3. An insertion device according to claim 1 or 2, characterized in that the bearing slider (47) axially displaceable in relation to the retaining head (3) and to the control slider (44) is spring biased (helical compression spring 64) counter to the driving direction (M) of the driver (5, 6).

4. An insertion device according to claim 3, characterized in that the driving link arrangement comprises, on the one hand, an elongated driving link (62) provided in each driving lever (45, 46) and extending at an acute angle (W) relative to the longitudinal axis (A) of the leading-in tube (1), and on the other hand, an elongated driving link (62') provided in the bearing slider (47) and extending axial-parallel, a control bolt (67) disposed in the control slider (44) engaging with the driving links (62, 62').

5. An insertion device according to one of the preceding claims, characterized in that the driving link arrangement comprises an elongated driving link (50) of axial-parallel extension, which is provided in the control slider (44) and through which a pivot bolt (66) passes for pivotally supporting the driving levers (45, 46) on the bearing slider (47).

6. An insertion device according to one of the preceding claims, characterized in that the control slider (44) is integrally molded on the distal end (43) of the push bar (8).

7. An insertion device according to one of the preceding claims, characterized in that the bearing slider (47) is forked, the control slider (44) and the driving levers (45, 46) being disposed between the two fork legs (57, 58).

8. An insertion device according to one of the preceding claims, characterized in that the retaining head (3) has a cap-shaped head casing (37), which comprises an axial-parallel, cylindrical bearing bore (40) having axialparallel, radially opposed wall openings (41, 42) for receiving the control slider (44), the bearing slider (47) and the driving levers (45, 46).

## Revendications

1. Appareil chirurgical pour la pose d'un anneau d'anastomose comportant :
- un tube d'introduction (1) pouvant être introduit dans les voies intestinales
- une tête de retenue (3) cylindrique disposée sur l'extrémité (2) corporelle interne du tube d'introduction (1) pour un anneau d'anastomose (4)
- un mécanisme pousseur disposé dans la région de la tête de retenue (3) pour la poussée conjointe et le blocage de l'anneau d'anastomose (4), mécanisme qui est actionnable depuis d'extrémité (7) corporelle externe du tube d'introduction (1) par l'intermédiaire d'un engrenage (9), et
- un entraînement (16) disposé sur l'extrémité (7) corporelle externe du tube d'introduction (1) pour l'engrenage (9) du mécanisme de poussée, le mécanisme de poussée présentant au moins un élément d'entraînement (5, 6) qui d'une part, est déplaçable axialement en deux étapes d'actionnement séparées à partir d'une position rétractée dans la tête de retenue (3) à une position en saillie latéralement à partir de la tête de retenue (3) radialement et d'autre part, dans sa position en saillie en entraînant l'anneau d'anostomose (4) à la position bloquée de celui-ci,
**caractérisé en ce que** respectivement un élément d'entraînement (5, 6) est disposé sur deux leviers d'entraînement basculant radialement dans la direction direction opposée de la tête de retenue (3) et qui sont déplaçables axialement longitudinalement (45, 46) et sont logés de façon pivotante dans un poussoir d'appui (47) les leviers d'entraînement (45, 46) et le poussoir d'appui (47) étant accouplés avec un poussoir de commande (44) raccordé à l'engrenage (9) par l'intermédiaire d'un agencement de coulisses de commande (50, 62, 62').

2. Appareil chirurgical pour la pose d'un anneau d'anastomose selon la revendication 1, caractérisé en ce que l'entraînement et l'engrenage pour le mécanisme de poussée sont conçus sous forme d'entraînement à broche (9) avec une roue à broche (16) fixée longitudinalement axialement par rapport à l'axe tubulaire (axe longitudinal A) logée de façon rotative coaxialement par rapport à celui-ci et une tige de poussée (8) déplaçable longitudinalement axialement dans le tube d'introduction (1) et bloquée dans la direction de rotation, tige de poussée (8) qui se trouve en prise avec un filetage interne (24) de la roue à broche (16) avec un filetage extérieur (26) sur son extrémité extérieure (27) et qui, par son extrémité corporellement interne (43) est accouplée avec le mécanisme de poussée.

3. Appareil chirurgical pour la pose d'un anneau d'anastomose selon la revendication 1 ou 2, caractérisé en ce que le dispositif de poussée déplaçable par rapport à la tête de retenue (3) et au poussoir de commande (44) est précontraint par un ressort (ressort hélicoïdal 64) en opposition à la direction d'entraînement (M) de l'élément d'entraînement (5, 6).

4. Appareil chirurgical pour la pose d'un anneau d'anastomose selon la revendication 3, caractérisé en ce que l'agencement de coulisses de commande comporte d'une part, une coulisse de commande (62) oblongue s étendant selon un angle aigu (W) par rapport à l'axe longitudinal (A) du tube d'introduction (1) dans chaque levier d'entraînement (45, 46) et d'autre part, une coulisse de commande oblongue s'étendant axialement parallèlement (62') dans poussoir d'appui (47), coulisses de commande (62, 62') dans lesquelles s'engage un boulon de commande (67) se situant dans le poussoir de commande (44).

5. Appareil chirurgical pour la pose d'un anneau d'anastomose selon l'une des revendications précitées, caractérisé en ce que l'agencement de coulisses de commande présente une coulisse de commande oblongue s'étendant axialement parallèlement dans le poussoir de commande (44), qui coopère avec le boulon basculant (66) pour le logement basculant des leviers d'entraînement (45, 46) sur le poussoir d'appui (47).

6. Appareil chirurgical pour la pose d'un anneau d'anastomose selon l'une des revendications précitées, caractérisé en ce que le poussoir de commande (44) est formé d'un seul tenant sur l'extrémité corporelle interne (43) de la tige de poussée (8).

7. Appareil chirurgical pour la pose d'un anneau d'anastomose selon l'une des revendications précitées, caractérisé en ce que le poussoir d'appui (47) a une conception en fourche entre les deux branches de fourche duquel (57, 58) sont agencés le poussoir de commande (44) et les leviers d'entraînement (45, 46).

8. Appareil chirurgical pour la pose d'un anneau d'anastomose selon l'une quelconque des revendications, caractérisé en ce que la tête de retenue (3) présente un logement de tête (37) en forme de capuchon qui présente un alésage cylindrique parallèle à l'axe (40) avec des ouvertures de paroi (41, 42) radialement en regard l'une de l'autre, parallèles à l'axe pour recevoir le poussoir de commande (44), le poussoir d'appui (47) et les leviers d'entraînement (45, 46).
